# EUROPEAN PATENT APPLICATION

(11) **EP 1 881 078 A1**
(43) Date of publication of application: **23.01.2008**
(21) Application number: 06730604.3
(22) Date of filing: 30.03.2006
(51) Int. Cl.: C12P 19/02, C12P 17/18, C12R 1/645, C12R 1/85

(54) **METHOD FOR PRODUCING GLUCURONIC ACID AND/OR GLUCURONOLACTONE**

(30) Priority: 11.05.2005 JP 2005138306
(71) Applicant: Ensuiko Sugar Refining Company, Limited, Chuo-ku Tokyo, 103-0012 (JP)
(72) Inventor: HAMAYASU, Kenichi, Ensuiko Sugar Refining Co., Ltd., Kanazawa-ku Yokohama-shi, Osaka 598-006 (JP); TADOKORO, Hiroki, Kansai Sugar Co., Ltd., Izumisano-shi, Osaka 5980061 (JP); KISHINO, Eriko, Ensuiko Sugar Refining Co. Ltd., Yokohama-shi, Kanagawa (JP); ITO,Tetsuya, Ensuiko Sugar Refining Co., Ltd., Yokohama-shi, Kanagawa (JP); FUJITA, Koki, Ensuiko Sugar Refining Co., Ltd., Yokohama-shi, Kanagawa (JP); HARA, Kozo, Ensuiko Sugar Refining Co., Ltd., Yokohama-shi, Kanagawa (JP)
(74) Representative: Gille Hrabal Struck Neidlein Prop Roos
(86) International application number: PCT/JP2006/306656
(87) International publication number: WO 2006/120813

(57) **Abstract**

Provided is a process, by which glucuronic acid and/or glucuronolactone can be produced in high yield, at low cost, in an easy manner and in safe. Also provided is a process for production of glucuronic acid and/or glucuronolactone which is **characterized in that** sucrose is oxidized to give sucrose carboxylic acid (and a salt thereof) (glucuronyl-fructoside, β-D-fructosyl- (2→1)-α-D-glucuronic acid and a salt thereof), yeast is added so as to hydrolyze fructose residue of the sucrose carboxylic acid and to assimilate the resulting product, and thus produced glucuronic acid and/or glucuronolactone are/is collected.

## Description

### Technical field

The present invention relates to a process for the production of glucuronic acid and/or glucuronolactone. More particularly, it relates to a process for the production of glucuronic acid and/or glucuronolactone which is characterized in that sucrose is oxidized to give sucrose carboxylic acid (and a salt thereof) (glucuronyl-fructoside, β-D-fructosyl- (2→1) - α-D-glucuronic acid and a salt thereof), then microorganism such as yeast is added so as to hydrolyze the fructose residue and to assimilate the resulting product, and thus obtained glucuronic acid and/or glucuronolactone are/is collected.

### Background art

Glucuronic acid and glucuronolactone or derivatives thereof have been widely used as pharmaceuticals and raw material for pharmaceuticals. Various synthetic methods for glucuronic acid and glucuronolactone have been known. An example of the common method is a process where glucose derivative or glucose derivative such as starch is used as a material, oxidized with a nitrogen compound such as nitric acid and then hydrolyzed to give glucuronic acid and glucuronolactone (refer to Patent Document 1 and 2).

Although the abovementioned process is a synthetic method which is able to be carried out in an industrial scale, a nitrogen oxide used as an oxidizing agent is relatively at high cost. In addition, the by-produced nitrogen monoxide gas bubbles and the scale tends to be very large and, moreover, the by-produced gas is a cause of environmental pollution. Therefore, at this time when environmental affairs have been serious, there is a problem that an apparatus for its recovery is necessary. There are some inventions for improvement thereof and, although they solve the problem of by-produced gas, they still have a problem in enhancement of the yield that has not been solved yet (refer to Patent Documents 3 and 4).

There is another method where C-1 position of glucose is protected, then C-6 position is oxidized and hydrolyzed and deprotection is then conducted to prepare glucuronic acid and glucuronolactone. The method has a problem that a step for deprotection is complicated (refer to Patent Document 5).

There is still another method where a selective oxidation using 6,6-tetramethylpiperidine N-oxyl (TEMPO) which is an oxidizing catalyst is carried out (refer to Patent Document 6). Although a conversion rate is high in this method, the preferred raw material is a monosaccharide derivative such as methyl glucoside in which C-1 position of glucose is protected. In addition, cost for the production of an amine oxide represented by TEMPO is high and, moreover, such an oxide has a possibility of giving a bad affection to human body.

As a means for solving the above, there is a proposal for a process which is characterized in that an adsorptive resin is used and is made to react with a halogen-containing oxide or an electrolytic oxide of halogen-containing compound together with an amine oxide which is a catalyst (refer to Patent Document 7).
In this method however, although the conversion rate of a glucose derivative to uronic acid is high and recovery of the catalyst is easy, the catalyst is still expensive. In addition, although the glucose derivative used as a raw material includes sucrose, the preferred ones are methyl-α-glucoside and isopropyl-α- and β-glucosides which are relatively expensive. Moreover, sodium hypochlorite or the like used as a material generates halogenic acid and is unable to be a so safe material. In the synthesis of glucuronolactone from the resulting glucuronic acid derivative such as methyl-α-glucopyranosidouronic acid, a step of subjecting to a conventional hydrolyzing reaction is necessary.

On the other hand, there is a disclosure for a method where trehalose carboxylic acid (α-D-glucuronyl α-D-glucuronate) is prepared from trehalose as a material using an oxidizing catalyst such as platinum oxide, palladium-carbon or platinum-carbon and is hydrolyzed using an acid or an enzyme to prepare an aimed glucuronic acid and glucuronolactone (refer to Patent Document 8).

In this method, yield from the material is good but amount of the oxidizing catalyst necessary therefor is much and cost becomes high. In addition, since a glucoside bond of a trehalose carboxylic acid which is an intermediate is stable, glucuronic acid is unable to be liberated unless a relatively aggressive hydrolysis with acid is carried out. There is another method where enzyme such as α-glucuronidase is used but the price therefor is usually expensive and, further, a hydrolyzing ability thereof is not high.

Patent Document 1: JP-B-46-3871
Patent Document 2: British Patent No. 900,977
Patent Document 3: JP-B-42-2595
Patent Document 4: JP-B-43-5882
Patent Document 5: JP-B-44-7325
Patent Document 6: JP-A-2-107790
Patent Document 7: JP-A-11-147043
Patent Document 8: JP-A-10-251263

### Disclosure of the invention

### Problem to be solved by the invention

Under such circumstances, an object of the present is to provide a process by which glucuronic acid and/or glucuronolactone are/is able to be produced in high yield, at low cost, in an easy manner and in safe.

### Means for solving the problem

As a result of intensive studies, the present inventors have found that aimed glucuronic acid and/or glucuronolactone are/is able to be produced in high yield when sucrose carboxylic acid (and/or a salt thereof) (glucuronyl-fructoside) are/is prepared using sucrose as a material and made to act to a microorganism having an invertase activity such as yeast.
During its production steps, nitrogen oxide or the like such as nitric acid is not used as an oxidizing agent or, even if used, its amount is little and, therefore, an aimed product is able to be produced safely without affecting on environment.
The present inventors have also investigated in the adding amount of yeast, etc., reaction condition and method for desalting and crystallizing and have found a production process which is easier and more effective than the conventional methods whereupon the present invention has been achieved.

The present invention mentioned in a first aspect of the present invention is a process for the production of glucuronic acid and/or glucuronolactone, characterized in that, sucrose is oxidized to give sucrose carboxylic acid or a salt thereof, then microorganism having an invertase activity is added so as to hydrolyze the fructose residue of the sucrose carboxylic acid or a salt thereof and to assimilate the resulting product, and thus obtained glucuronic acid and/or glucuronolactone are/is collected.
The present invention mentioned in a second aspect of the present invention is a production process according to the first aspect, wherein the microorganism having an invertase activity is yeast.
The present invention mentioned in a third aspect of the present invention is a production process according to the first aspect, wherein sucrose is selected from the group of pure sugar, fine liquor, raw sugar, beet sugar, maple syrup, sugar-added preparation or waste molasses.
The present invention mentioned in a fourth aspect of the present invention is a production process according to the first aspect, wherein the reaction where the microorganism having an invertase activity is added to sucrose carboxylic acid or a salt thereof to hydrolyze the fructose residue of the sucrose carboxylic acid or a salt thereof is carried out in such a manner that the microorganism is permitted to act on an aqueous solution containing sucrose carboxylic acid or a salt thereof in a concentration of 1 to 50% at the temperature of 0 to 60°C and at pH 3 to 10.

### Effect of the invention

In accordance with the present invention, it is now possible to prepare aimed glucuronic acid and/or glucuronolactone, not only using sucrose which is a less expensive material, but also in a significantly high yield.
In addition, as mentioned before, nitrogen oxide or the like such as nitric acid is not used as an oxidizing agent or, even if used, its amount is very little during its production steps and, therefore, the aimed product is able to be produced safely and without affecting the environment. Moreover, investment in plant and equipment is relatively small as well.
Consequently, a process for the production of glucuronic acid and/or glucuronolactone according to the present invention is an industrial method having a good productivity as compared with the conventional methods.

### Brief description of the drawings

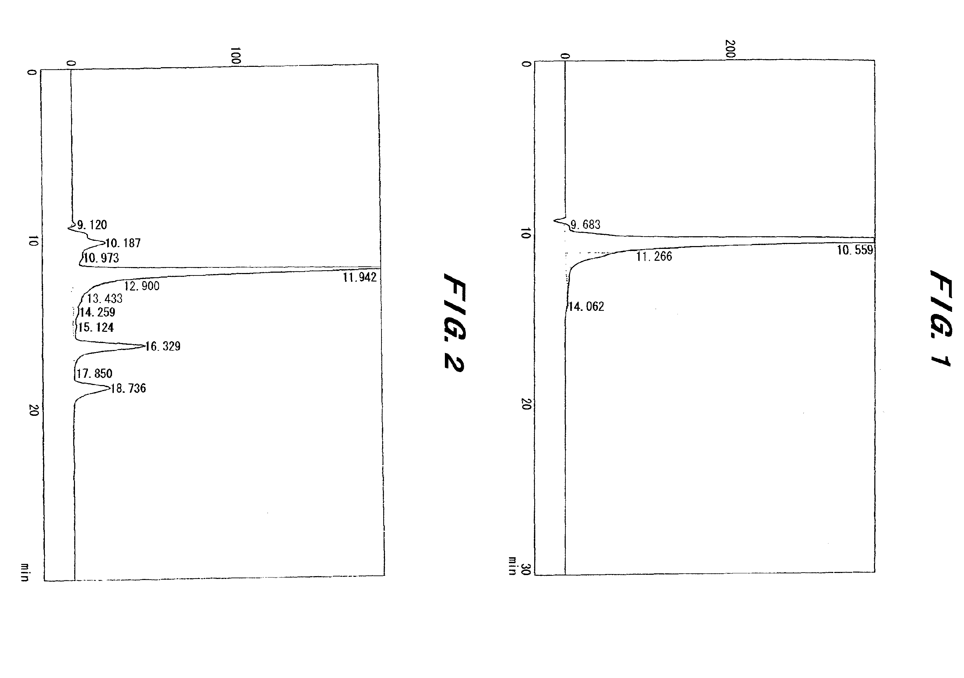

[Fig. 1] shows an HPLC chromatogram of sodium sucrose carboxylate.
[Fig. 2] shows an HPLC chromatogram of desalted solution for crystallizing of glucuronic acid and/or glucuronolactone after assimilation of sodium sucrose carboxylate with yeast.

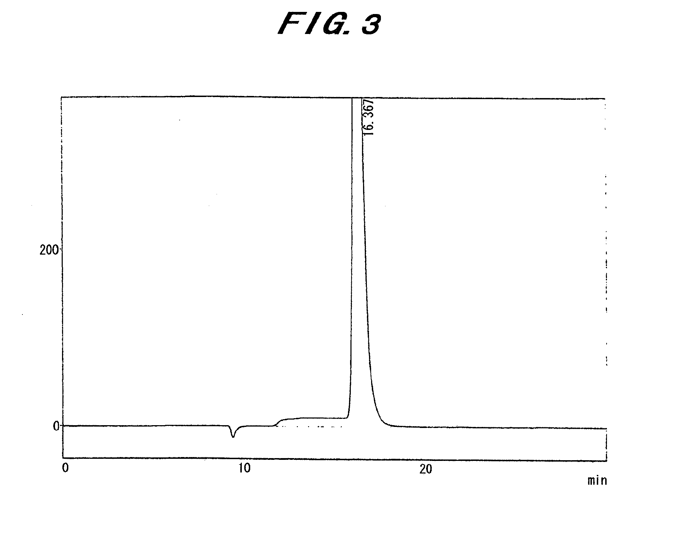

[Fig. 3] shows an HPLC chromatogram of the resulting glucuronolactone.
The best embodiment for carrying out the invention

The present invention is mentioned in detail as follows.
A series of reaction in the process for production of glucuronic acid and/or glucuronolactone according to the present invention is as shown in the following formulae.

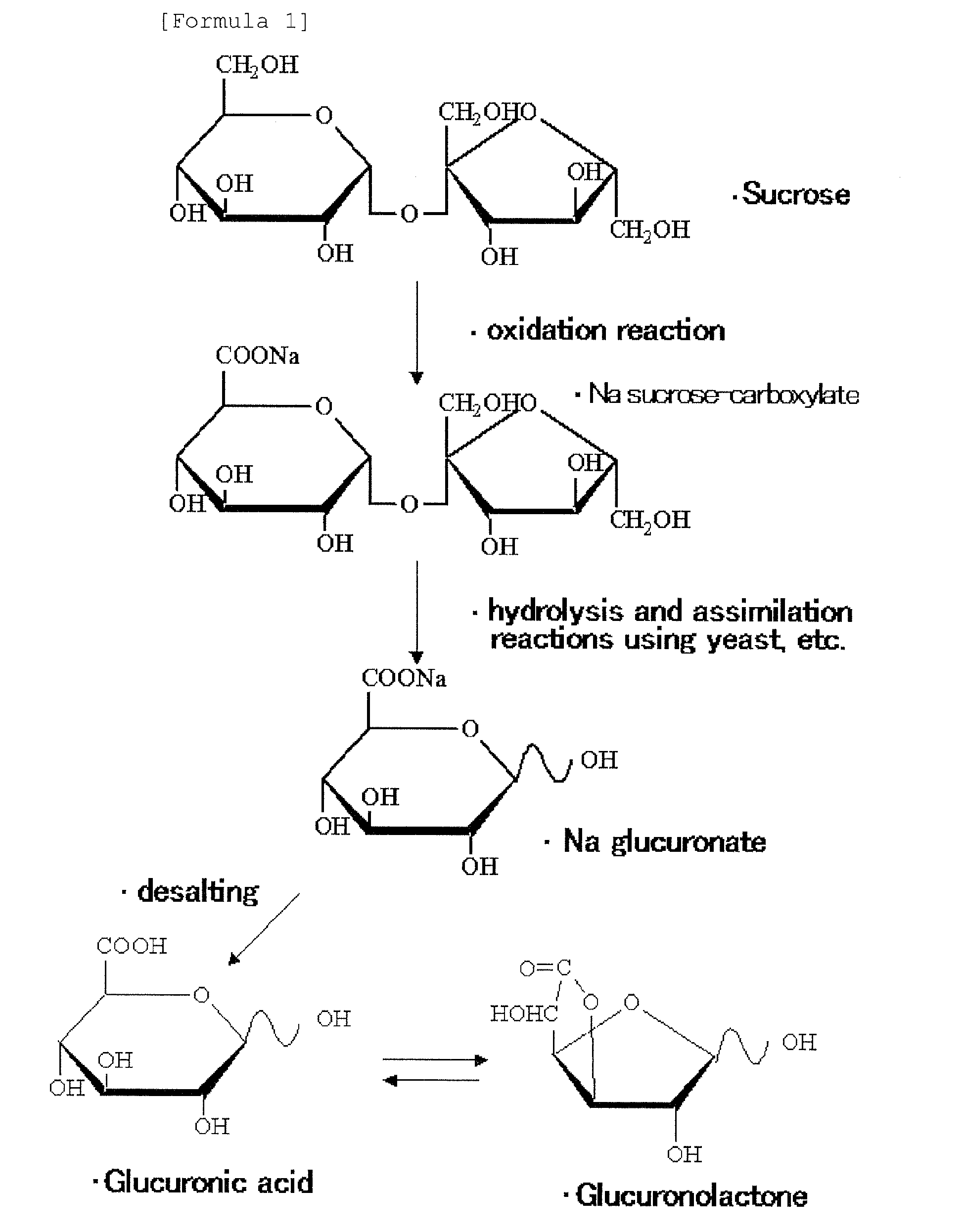

Sucrose (chemical formula: C₁₂H₂₂O₁₁) used in the present invention is a non-reducing disaccharide synthesized by plants in photosynthesis. It is usually manufactured by repeated purification and crystallization of extracted juice of sugar cane or sugar beet.
In the present invention, there is no particular limitation for the origin of sucrose. Although it is preferred to be pure sugar and is also fine liquor, raw sugar, beet sugar, maple syrup, etc., anything may be used so far as it contains sucrose and its examples are sucrose-containing substances such as waste molasses, sugar-added preparations and fructo-oligosaccharide which may be appropriately selected after investigating the cost and the production process.

With regard to a method for oxidization of sucrose, conventional method is utilized. Thus, an inorganic nitrogen compound such as nitric acid, nitrous acid and salts thereof, a metal compound such as compound of manganese, chromium or lead and others such as halogen, ozone and oxygen may be used as an oxidizing agent. It is also possible to use platinum oxide, platinum-carbon, vanadium oxide, palladium-carbon, etc. as a catalyst but, in such a method, a by-product may be generated.
In view of the above, it is preferred, as shown in Patent Document 7, that an electrolytically oxidized halogen-containing compound is used together with resin with which an amine oxide is adsorbed whereupon a primary hydroxyl group of the glucose residue of the sucrose is selectively oxidized to prepare a glucuronyl-fructoside.

The most effective method is, as shown in Japanese Patent No. 3,556,690, to conduct an oxidative fermentation using a microorganism such as *Pseudogluconobacter saccharoketogenes.*
In this method, a primary hydroxyl group of the glucose residue of the sucrose is selectively oxidized whereby glucuronyl-fructoside (β-D-fructosyl-(2→1)-α-D-glucuronic acid) and a salt thereof is able to be produced. Thus, as mentioned in the Examples, the microorganism, culture liquid of the microorganism or enzyme produced by the microorganism is made to act whereupon sucrose carboxylic acid (glucuronyl-fructoside) (and a salt thereof) is able to be produced. Since fermentation using a microorganism or oxidation reaction using an enzyme is able to be carried out under a mild condition, an aimed product is able to be produced in a safe manner and with little influence on environment. In addition, investment for plant and equipment is relatively small as well.

Anyway, it is acceptable provided that a reaction solution containing sucrose carboxylic acid (and a salt thereof) (glucuronyl-fructoside) is able to be produced by a selective oxidation of a primary hydroxyl group of a glucose residue of sucrose.

In order to produce the aimed glucuronic acid or glucuronolactone, the sucrose carboxylic acid or a salt thereof (glucuronyl-fructoside) prepared by the aforementioned method is made to act on a microorganism having an invertase activity such as yeast. Yeast or the like hydrolyzes and also assimilates the fructose residue of glucuronyl-fructoside. As a result, the aimed glucuronic acid or glucuronolactone is able to be prepared.

The condition when yeast or the like is made to act on sucrose carboxylic acid or a salt thereof (glucuronyl-fructoside) may be free so far as it is a condition where yeast or the like is able to hydrolyze and also assimilate the fructose residue of glucuronyl-fructoside.
In the above reaction, water is preferred as a solvent which dissolves sucrose carboxylic acid or a salt thereof (glucuronyl-fructoside).

In the above-mentioned reaction, concentration of sucrose carboxylic acid or a salt thereof (glucuronyl-fructoside) is usually 1 to 50% and, preferably, 5 to 30%.
Temperature for the above-mentioned reaction is usually within a range of 0 to 60°C and, preferably, 15 to 40°C although the temperature is not limited thereto so far as it is a condition where yeast or the like is able to remove the fructose residue by means of hydrolysis and assimilation.

The pH for the above-mentioned reaction is usually 3 to 10 and preferably 4 to 8 and, since the optimum pH varies depending upon the type of the yeast or the like, the pH is not limited thereto so far as it is a condition where the yeast or the like used therefor is able to hydrolyze and assimilate the fructose residue.
In general, pH value tends to lower as the reaction proceeds but, within the above-mentioned range, it is not necessary to adjust the pH value. Incidentally, it is preferred to stir during the reaction whereby proliferation and assimilation of the yeast or the like may sometimes be accelerated.

The microorganism which is able to be used for hydrolysis of sucrose carboxylic acid or a salt is a microorganism having an invertase activity and yeast is particularly preferred. Examples of the type of yeast are genus *Saccharomyces* represented by bread yeast and yeast for the manufacture of Japanese sake as well as genus Candida, genus *Pichia* and genus *Schizosaccharomyces.* Besides them, even fungi or bacteria are able to be used in the present invention provided that they have an invertase activity and are able to assimilate a fructose residue.
In view of the safety of glucuronic acid and glucuronolactone produced by the present invention and also of the fact that those compounds are applied to the field of food, pharmaceuticals, etc., it is preferred to use yeast of genus *Saccharomyces* represented by bread yeast and yeast for the manufacture of Japanese sake as a microorganism. It is more preferred to use the bread yeast which is cheap and has a strong invertase activity.
Adding amount of the yeast or the like to a reaction system varies depending upon various conditions such as reaction time, temperature, concentration and pH but, usually, it is 0.1 to 10% and, preferably, 1 to 5%.

Although the reaction time is usually 1 to 240 hour (s) and, preferably, 24 to 120 hours, it is not limited thereto provided that it is within a condition that yeast or the like is able to hydrolyze and assimilate the fructose residue.
Incidentally, it is also possible to use the yeast or the like after being immobilized using an already-reported method or the like such as calcium alginate and that is a preferred embodiment because, if it is able to be recovered after use by that, a reduction in the cost is possible.
When the reaction proceeds and fructose is able to be removed from the reaction system as a result of assimilation, yeast or the like is removed and concentration, decolorization and desalting are carried out.

Yeast assimilates hydrolyzed fructose and grows and, at the same time, it produces ethanol, carbon dioxide, glycerol, organic acid, etc. Among them, ethanol and carbon dioxide are able to be removed upon heating or concentrating. Ethanol is recovered and may be used separately.

Since the resulting reaction solution containing obtained glucuronic acid and/or glucuronolactone contains salt, organic acid, coloring agent, microorganism-derived protein, etc., they are removed using an acidic, basic or amphoteric resin. If desired, active carbon, electrodialysis apparatus, etc. may be used.

A decolorized and desalted solution containing glucuronic acid and/or glucuronolactone may be subjected to a common method for crystallization of sugar or sugar derivatives to prepare an aimed product. To be more specific, the solution is concentrated and then crystals of glucuronolactone or glucuronic acid are inoculated followed by crystallizing.
When temperature and time are applied, glucuronic acid and glucuronolactone usually reach equilibrium. Since glucuronolactone is crystallized more easily, it is usually recommended that concentration is adjusted so as to make the content of the solid 40 to 70%, crystals of glucuronolactone are inoculated and the aimed product is crystallized. On the other hand, when crystals of glucuronic acid are to be obtained, the solid content is concentrated up to 65% or more so that crystals of glucuronic acid are crystallized.
The aimed product is able to be recovered efficiently by the repetition of steps comprising; the solution containing crystals is isolated by means of centrifugal separation or the like, the filtrate is concentrated again and crystallization is repeated again.

As a result of a series of reactions as mentioned above, it is possible to produce an aimed product from sucrose as a raw material in a yield of as high as 20 to 40% by weight. In the present invention, less expensive sucrose is used as a raw material whereby the aimed product is able to be produced at low cost.
Further, when an oxidation reaction of sucrose is carried out using a microorganism as mentioned in Japanese Patent No. 3,556,690, it is possible to produce the aimed product by adoption of a mild condition throughout all steps whereby bad influences on environment is also little.

Glucuronic acid and/or glucuronolactone produced by the production process of the present invention have/has the same or even better purity as compared with the product(s) prepared by known methods. Accordingly, they/it are/is widely used in various fields such as pharmaceutical industry, food industry, cosmetic industry and chemical industry as substance(s) having a recovering action for liver function, a recovering action from fatigue, an action for conjugation and detoxification and an anti-rheumatic action.

### Example 1

The present invention will now be illustrated by the following Examples in more detail although the present invention is not limited thereto.

### (1) Production of sodium sucrose carboxylate

From 360 kg of sucrose (granular sugar) was prepared a solution of sodium sucrose carboxylate (glucuronyl-fructoside) of the same amount using an oxidase-productive microorganism according to a method mentioned in Example 1 of Japanese Patent No. 3, 556, 690. Thus, 30 kg of sucrose and 250 L of water were added to a fermentation tank, the mixture was dissolved, 50 L of a washed cell suspension of *Pseudogluconobacter saccharoketogenes* which was separately prepared in a fermentation tank having the same capacity was added and the total volume was made 300 L. After that, the reaction was carried out at 32°C with stirring at 200 rpm under aeration at the rate of 100 L/minute for 24 hours to give the aimed sodium sucrose carboxylate. Then the cells were collected and reused depending upon the presence/absence of the activity and the same method was repeated 12 times to give a solution containing 360 kg of solid part of sodium sucrose carboxylate (glucuronyl-fructoside).

### (2) Decomposition of sodium sucrose monocarboxylate

The solid corresponding to 60 kg of sodium sucrose carboxylate prepared in the above (1) was concentrated to 30%, 3% (10.8 g) of bread yeast (FD-1, manufactured by Oriental Yeast Co., Ltd.) per solid part added at 37°C and a hydrolyzing reaction was carried out for 72 hours. No adjustment of the pH was conducted during the reaction, and the final pH was about 4. After completion of the hydrolysis, heating was conducted at 80°C for 5 minutes to sterilize and then a UF filtration was conducted to recover the filtrate. The filtrate was concentrated to 50% using a concentrating apparatus (Evapor, manufactured by Okawara MFG. Co., Ltd.) and ethanol (produced by assimilation due to the yeast of hydrolytic products) contained in the filtrate was recovered. The same method was repeated for six times to prepare a concentrate of sucrose carboxylic acid.

After that, the concentrate was passed, by dividing into six, through 50 L of active carbon column (Shirasagi, manufactured by Japan EnviroChemicals, Ltd.), 50 L of basic ion-exchange resin (Amberlite IRA-96SB, manufactured by Organo Corporation) and 150 L of strongly acidic ion-exchange resin (Diaion PK-216, manufactured by Mitsubishi Chemical Corporation) to decolorize and desalt.

### (3) Crystallization of glucuronolactone

The desalted solution prepared as such was concentrated to 50% by a concentrating apparatus (Evapor, manufactured by Okawara MFG. Co., Ltd.) to give a syrup for crystallization. The syrup for crystallization was transferred to a vacuum pan (crystallizing pan) (manufactured by Tsukishima Kikai Co., Ltd.) and concentrated to 62%, equilibrium in the ratio of glucuronic acid to glucuronolactone in the reaction solution was changed at about 45°C to increase the content of glucuronolactone, 50 g of seed crystals of glucuronolactone were added thereto and crystallization was carried out for one night and day under natural cooling.
On the next day, the crystals were made to grow by concentration under reduced pressure (68%, 42°C) and then temperature was raised up to 45 to 60°C. After that, fluidity of the solution was increased and the solution was transferred to a crystallizer (manufactured by Tsukishima Kikai Co., Ltd.) and cooled until the temperature reached 20°C at the rate of 2°C/hour. The resulting glucuronolactone crystals were recovered by a centrifugation (1,200 rpm), washed with 70% ethanol and dried at 50°C using a shelf dryer.

In the aforementioned operation, 28.1 kg of crystals were able to be prepared by single crystallizing step. The supernatant was recovered and subjected to the same crystallization whereupon 100 kg of glucuronolactone was able to be recovered.
Glycerol and unknown components that were produced during the assimilating step by the yeast in the syrup for crystallization were able to be easily removed by means of crystallization.
When purity of the resulting glucuronolactone was confirmed by HPLC (column: SCR-101H column manufactured by Shimadzu Corporation; mobile phase: 20 mM sulfuric acid; detection: RI; flow rate: 0.5 mL/min), it was 99.9% or more. Retention time in the HPLC thereof was the same as that of reagent grade glucuronolactone.

### Example 2

From 15 kg of sucrose, potassium sucrose carboxylate in the same amount was prepared according to the method mentioned in Example 1.
From the above, 100 g of potassium sucrose carboxylate was taken, 7 g of commercially available bread yeast (dry yeast) was added thereto and hydrolytic and assimilating reactions were carried out at 37°C.
As a result, sucrose carboxylic acid was hydrolyzed within 24 hours to give a solution containing glucuronic acid and glucuronolactone. After that, the solution was passed through 1 L of active carbon column (Shirasagi, manufactured by Japan EnviroChemicals, Ltd.), 1 L of basic ion-exchange resin (Amberlite IRA-96SB, manufactured by Organo Corporation) and 3 L of strongly acidic ion-exchange resin (Diaion PK-216, manufactured by Mitsubishi Chemical Corporation) to decolorize and desalt.

The desalted solution was concentrated to 65% at 40°C and 0.2 g of seed crystals of glucuronic acid were added thereto followed by subjecting to natural cooling. The crystals separated out therefrom were centrifuged to give 40 g of glucuronic acid.
When purity of the resulting glucuronic acid was confirmed by HPLC (column: SCR-101H column manufactured by Shimadzu Corporation; mobile phase: 20 mM sulfuric acid; detection: RI; flow rate: 0.5 mL/min), it was 99.9% or more.

### Example 3

From 15 kg of raw sucrose, sodium sucrose carboxylate in the same amount was prepared according to the method mentioned in Example 1.
After that, 15 kg of a sodium sucrose carboxylate solution was concentrated to 30% according to a method mentioned in Example 1 in the same manner, bread yeast (FD-1, manufactured by Oriental Yeast Co., Ltd.) was added at 3% per solid part and reaction was carried out at 37°C for 72 hours.
The reaction mixture was passed through an active carbon column and a desalting resin column similarly to decolorize and desalt. The resulting desalted solution was concentrated to 58% at 50°C and 20 g of seed crystals of glucuronolactone were added whereupon the crystals were separated out. After that, centrifugal separation was conducted to give 2 kg of glucuronolactone.

### Example 4

From 20 kg of beet sugar, sodium sucrose carboxylate in the same amount was prepared according to the method mentioned in Example 1. After that, 20 kg of a sodium sucrose carboxylate solution was concentrated to 30% according to a method mentioned in Example 1 in the same manner, bread yeast (FD-1, manufactured by Oriental Yeast Co., Ltd.) was added at 4% per solid part and reaction was carried out at 37°C for 72 hours.
The reaction mixture was passed through an active carbon column and a desalting resin column similarly to decolorize and desalt. The resulting desalted solution was concentrated to 58% at 50°C and 20 g of seed crystals of glucuronolactone were added whereupon the crystals were separated out. After that, centrifugal separation was conducted to give 4 kg of glucuronolactone. Industrial applicability

In accordance with the present invention, it is now possible to produce glucuronic acid and/or glucuronolactone in a high yield from sucrose which is a less expensive raw material.
In addition, no nitrogen oxide such as nitric acid is used as an oxidizing agent during the production steps or, even if used, its amount is very small and, therefore, the aimed product is able to be produced safely and without affecting the environment.

## Claims

1. A process for the production of glucuronic acid and/or glucuronolactone, **characterized in that**, sucrose is oxidized to give sucrose carboxylic acid or a salt thereof, then microorganism having an invertase activity is added so that the fructose residue of the sucrose carboxylic acid or a salt thereof is hydrolyzed , the resulting product is assimilated, and thus produced glucuronic acid and/or glucuronolactone are/is collected.

2. The production process according to claim 1, wherein the microorganism having an invertase activity is yeast.

3. The production process according to claim 1, wherein sucrose is selected from the group of pure sugar, fine liquor, raw sugar, beet sugar, maple syrup, sugar-added preparation or waste molasses.

4. The production process according to claim 1, wherein the reaction where the microorganism having an invertase activity is added to sucrose carboxylic acid or a salt thereof to hydrolyze the fructose residue of the sucrose carboxylic acid or a salt thereof is carried out in such a manner that the microorganism is permitted to act on an aqueous solution containing sucrose carboxylic acid or a salt thereof in a concentration of 1 to 50% at the temperature of 0 to 60°C and at pH 3 to 10.
